# FASCICULE DE BREVET EUROPEEN

(11) **EP 2 755 498 B1**
(45) Date de publication et mention de la délivrance du brevet: **07.02.2018**
(21) Numéro de dépôt: 12753484.0
(22) Date de dépôt: 31.08.2012
(51) Int. Cl.: A23K 20/00, A23K 20/10, A23K 40/20, A23K 50/20, A23K 50/42

(54) **COMPOSITION POUR ADMINISTRATION ORALE AUX ANIMAUX ET PROCEDE D'OBTENTION**
ZUSAMMENSETZUNG ZUR ORALEN VERABREICHUNG AN TIERE UND DEREN HERSTELLUNGSVERFAHREN
COMPOSITION FOR ORAL ADMINISTRATION TO ANIMALS AND PRODUCTION METHOD

(30) Priorité: 15.09.2011 WO PCT/EP2011/065990
(43) Date de publication de la demande: 23.07.2014
(73) Titulaire: Friulchem SpA, 33099 Vivaro (IT)
(72) Inventeur: DERRIEU, Guy, F-06800 Cagnes-sur-Mer (FR); MAZZOLA, Giancarlo, CH-6930 Bedano (CH)
(74) Mandataire: Regimbeau
(86) Numéro de dépôt international: PCT/EP2012/067005
(87) Numéro de publication internationale: WO 2013/037650

(56) Documents cités:
- WO-A1-2009/068378
- US-A1- 2004 151 759
- US-A1- 2005 226 908
- US-A1- 2007 128 251
- US-A1- 2008 160 067
- US-A1- 2008 166 459
- US-A1- 2011 183 036

## Description

La présente invention se rapporte au domaine de la préparation de compositions appétissantes à visée thérapeutique plus particulièrement pour améliorer la prise orale et garantir le traitement, formulées sous une forme solide, par les animaux (domestiques, d'élevage ou sauvages).

On assiste actuellement à une évolution des soins qui sont prodigués aux animaux, et la voie orale devient une voie privilégiée pour l'administration de médicaments par le professionnel de santé ou par le propriétaire, et cela est plus particulièrement vrai pour les produits à visée nutraceutique. En effet, les voies d'administration parentérales usuelles (intramusculaire, sous-cutanée, intradermique ou intraveineuse) en particulier des médicaments présentent certains inconvénients. Les voies intramusculaire ou sous-cutanée peuvent, par exemple, être à l'origine d'hématomes ou d'abcès. La voie intraveineuse requiert souvent l'intervention d'un spécialiste (vétérinaire). Quant à la voie intradermique, elle nécessite l'emploi de solvants pour faire passer la barrière cutanée aux molécules actives. Ces voies d'administration parentérales nécessitent également la contention des animaux. Par ailleurs, certaines substances actives sont difficiles à formuler dans des formes galéniques parentérales. Enfin, certaines substances actives n'exerceront leur action thérapeutique chez l'animal que si elles arrivent directement dans l'appareil digestif. Pour avoir la prise de la composition par l'animal il faut qu'il soit attiré et qu'il absorbe cette dernière on notera alors la satisfaction de l'animal qui se manifestera par une sollicitation de l'animal afin d'obtenir une autre composition. C'est cette satisfaction qui fera le plus grand plaisir au propriétaire de l'animal et qu'il recherche.

Il est connu que l'acceptation et la consommation naturelles par un animal d'une composition repose sur sa forme galénique principalement sur deux caractéristiques, l'appétence et la texture de ladite composition, et avec une moindre importance la forme et la taille de cette dernière.

A ces paramètres il faut en ajouter deux autres :
- un parfait contrôle de la quantité d'une ou des substances actives aussi bien si la composition est un médicament ou un nutraceutique ou complément alimentaire, et
- le coût de la composition particulièrement si celle-ci est un nutraceutique ou un complément alimentaire.

Les formulations galéniques adaptées à l'administration de compositions par voie orale ou *per os* se présentent généralement sous forme liquide (tels que des sirops, des solutions ou des suspensions buvables, des gouttes...), sous forme semi-solide (telle que les pâtes pour administration orale) ou sous forme solide. Les formes solides couramment utilisées *per* os dans le règne animal, se présentent sous diverses formulations de nature différente et sont obtenues par différents procédés. On distingue par exemple les comprimés, les cachets, les capsules, les gélules, les gommes à mâcher, les pilules, les pastilles, les tablettes. Principalement pour l'observance des traitements administrés par voie orale (c'est-à-dire le respect des consignes et directives du professionnel de santé concernant la prise des médicaments), on constate que le traitement n'est pas toujours convenablement suivi, en raison de la difficulté à administrer en totalité des traitements aux animaux. En effet, l'administration aux animaux de médicaments sous forme galénique solide par voie orale est souvent difficile du fait du mauvais goût de certaines substances actives ou de certains excipients constituant le médicament et du sens très développé de l'odorat et du goût des animaux. On a observé, chez les animaux, que la principale raison qui rend très difficile, voire impossible, l'observance d'un traitement oral est le défaut d'appétence suscitée par le médicament. Il en est de même avec des nutraceutiques ou compléments alimentaires. En effet, un propriétaire est particulièrement sensible aux réactions de son animal lorsqu'il s'agit de prendre une composition. Une composition donnée à un animal doit être un plaisir que partage le propriétaire avec son animal.

L'appétence se définit comme l'état psychologique correspondant à un désir d'absorber un aliment ou une boisson en réponse à la perception des caractères organoleptiques de ce produit. La capacité à susciter l'appétence est appelée appétissance. La combinaison de ces caractères détermine l'attrait qu'aura un produit à prendre par la voie orale sur des animaux normalement nourris. Plus particulièrement l'appétissance d'un médicament participe grandement au refus ou à l'acceptation par l'animal de la prise spontanée du traitement et de la répétition de la prise sur des périodes parfois longues. Dans le cadre de certains traitements, la prise du traitement peut être journalière voire à vie.

L'appétissance d'un médicament, d'un nutraceutique ou complément alimentaire administré par voie orale conduit à l'acceptation et à l'ingestion volontaire par les animaux. Cette appétissance peut être mesurée dans un essai général d'appétence prenant en compte différents paramètres de la composition formulée sous une forme solide, comme sa prise spontanée à la main ou au sol, ou encore sa consommation, même si elle est donnée en plusieurs fois ou prise à intervalles réguliers par l'animal.

La texture se définit comme un état physique correspondant à une formulation agencée d'une certaine manière par une technologie d'obtention. C'est de la texture que dépendent la dureté, la friabilité, la mollesse, l'élasticité, la couleur de la composition.

Quant aux paramètres, forme ou taille, ils vont faciliter la préhension de la composition ou son absorption en une seule fois.

Dans l'art antérieur, de nombreuses solutions combinant ou non ces deux paramètres majeurs voire les autres ont été proposées pour faciliter l'absorption principalement des médicaments par un animal.

Concernant l'appétence, plusieurs voies ont été proposées :
Une première option consiste à masquer le goût et/ou l'odeur désagréable du ou des constituants, principalement une ou des substances actives, par encapsulation ou par enrobage de ces dernières.

Les demandes de brevet suivantes: EP 0 997 143, EP 1 490 037, WO 01/15547, AU 2001279664, FR 2 350 105, US 5,380,535, US 3,037,911, décrivent comment encapsuler ou enrober un ou des constituants ainsi que les techniques pour y arriver.

Ces solutions nécessitent de nombreuses étapes d'encapsulation ou d'enrobage ou elles font intervenir une étape de production comme l'extrusion qui est susceptible de dégrader les substances actives fragiles ou dénaturer les constituants comme les arômes ou les matières appétibles.

Selon une autre option pour faciliter l'administration orale est d'englober la composition, principalement le médicament, dans des matières appétibles.

Les demandes de brevet suivantes: FR 2 896 958, FR 2 715 803, US 5, 853, 757, US 6,143, 316, US 5, 792, 470, US 5, 674, 515, EP 0 574 301, US 4, 857, 333, DE 198 53 729, WO 03/030863 , WO 2004/043427, WO 2007/090987, proposent des leurres réalisés avec des matières appétibles.

L'inconvénient de ces leurres est que leur utilisation nécessite une manipulation à savoir l'introduction du médicament au préalable dans le leurre, ce qui peut rebuter certains utilisateurs et aussi devenir gênant lorsqu'un grand nombre d'animaux doit être traité. De plus, leur volume important (nécessairement plus gros que le médicament) demande une quantité de matière importante et leur fabrication doit être adaptée à leur forme complexe ; ainsi ces leurres s'avèrent souvent coûteux.

Encore une autre option, les substances actives sont isolés au milieu d'une matrice obtenu par compression des constituants secs, comprenant une substance appétible afin de masquer leur goût et faciliter leur prise et leur consommation.

La demande de brevet EP 0 320 320 décrit un comprimé pour animal domestique caractérisé en ce qu'il est constitué par au moins un noyau contenant une ou plusieurs substances actives totalement englobées dans une matrice appétible pour l'animal. Dans de telles compositions c'est la texture qui constitue l'inconvénient majeur. En effet la forme comprimé ou tablette donne des formes dures peu appréciées des animaux. De plus, il faut une grande rigueur dans la fabrication de ce type de comprimés à noyau pour être sûr d'englober parfaitement la partie centrale.

Les demandes de brevet suivantes EP 0 725 570, EP 0 725 627, proposent des compositions formées de deux parties, l'une centrale renfermant les constituants au goût et/ou à l'odeur désagréable, l'autre extérieure englobant la partie centrale. Ceux sont des appâts et leur objectif est d'attirer l'animal pour qu'il le morde afin qu'il soit vacciné. Plusieurs inconvénients apparaissent :
- à la réalisation de la matrice, une augmentation de la température, pour obtenir la fusion de certains constituants est nécessaire pour fondre le polymère afin qu'il soit intimement mélangé, cela peut constituer une forte contrainte pour la stabilité de la substance active ,
- la réalisation de telles matrices est complexe et coûteuse,
- une conservation dans des conditions particulières pour garantir l'intégrité de la forme galénique.

La demande de brevet FR 2 709 420 décrit une forme et une taille pour un comprimé afin qu'il soit plus préhensible par les animaux en particulier les chats.

Les demandes de brevet suivantes : IE 2004 0393, et GB 2 432 506US, décrivent des objets à mâcher appétibles, obtenus par extrusion ou extrusion moulage. Le principal inconvénient de ces objets est qu'ils sont consommés pendant plusieurs minutes voire abandonnés et repris pour être remâchés avant d'être consommés. Il n'est donc pas possible de suivre un schéma thérapeutique avec certitude.

La demande de brevet US 2011/0183036 décrit des récompenses appétibles, obtenues par extrusion-moulage. Le principal inconvénient de ces récompenses est qu'elles sont obtenues à chaud, température supérieure à 82°C, à partir d'une pâte extrudée-moulée donc un manque de régularité du poids. De plus, cette pâte contient de l'eau dont une partie, entre 3 et 15%, reste dans la récompense après sa conformation. La demande parle bien d'une récompense et pas d'un objet médicamenteux.

Le brevet US 6,455,083) décrit des objets à mâcher comestibles, obtenus par extrusion-moulage. Le principal inconvénient est qu'ils sont des thermoplastiques c'est-à-dire que, dans l'extrudeur, il y a une polymérisation principalement des protéines avec l'eau (10 à 20%). Compte tenu de leur texture ces objets ne seront pas absorbées d'un seul coup mais mâcher au risque d'être abandonnés. Le brevet décrit bien des objets à mâcher nutritionnels et pas d'objets médicamenteux.

Les demandes de brevet suivantes : FR 2 154 424, et US 5,894,029 se réfèrent à la production d'aliments pour animaux domestiques. Les procédés de production mis en oeuvre ne sont nullement adaptés à la production de médicament particulièrement. Ils peuvent nuire grandement à la stabilité des substances actives par l'utilisation d'eau dans les formules ou les procédés, par l'utilisation de la chaleur avec ou non de la pression.

La demande de brevet US 5,637,313 décrit des compositions particulières mais surtout un procédé d'obtention particulier pour obtenir des comprimés enrobés à savoir la matrice après mélange est roulée en cordon d'un certain diamètre et coupée à une certaine longueur pour obtenir le poids désiré. Cette production ne permet pas de garantir un contrôle parfait du poids donc de la quantité de substance active par unité ce qui est antinomique avec un médicament.

Concernant la texture sachant qu'elle est fortement liée à la composition donc aussi à l'appétence trois procédés d'obtention ont été principalement proposés :
Le premier, le plus ancien, la compression à sec donnant des comprimés, des tablettes ou des pilules. Les demandes de brevets déjà citées : EP 0 997 143, EP 1 490 037, EP 0 574 301, EP 0 320 320, US 5, 380, 535, US 3,037,911, FR 2 709 420 décrivent des formes sèches dures peu appréciées des animaux et de nombreux refus par les animaux sont signalés.

Le deuxième, plus récent, est la production de tablettes à mâcher obtenues par extrusion Ainsi les demandes de brevet suivantes : U S 2004/0043925, US 2004/0037869, US 2001/036464, WO 2008/030469 , WO 2005/013714, WO 89/12442, décrivent des formes appétissantes extrudées ayant une texture relativement molle très appréciée des animaux en particulier les animaux domestiques comme le chien et le chat. Compte tenu du mode de production à savoir l'utilisation d'un extrudeur, il y a une sortie en ligne donc en continu de la matrice qui est ensuite coupée à la longueur désirée pour avoir le poids requis donc la quantité constante de substance(s) active(s) pour être en particulier un médicament. Cela demande un parfait contrôle des paramètres d'extrusion. Malheureusement nous savons qu'avec cette technique nous ne sommes pas à l'abri d'une variation de densité de l'extrudat qui conduit inévitablement à longueur constante à une variation du poids. Pour palier cela il faut peser une à une les tablettes à mâcher et écarter les hors normes qui ne pourront pas être recyclées sous peine de voir la mise à disposition de la substance active modifiée par une nouvelle extrusion. Cette technique s'avère nullement économique. En conclusion elle ne serait pas retenue par l'Homme de l'Art pour faire un médicament ou un nutraceutique ou complément alimentaire où la quantité de substance(s) active(s) doit être parfaitement contrôlée.

La demande de brevet US 2004/151759 décrit des comprimés à mâcher obtenus par des techniques conventionnelles, la compression de poudre granulée sèche ou extrusion avec un séchage à 50°C. Les deux procédés de fabrication font intervenir l'eau pour effectuer la granulation, eau éliminée avant la compression pour obtenir des comprimés classiques ou après l'extrusion pour obtenir des comprimés à mâcher. Les inconvénients des deux techniques qui ont déjà été évoquées sont présents.

Le troisième, dernier proposé, est la production de tablettes molles par moulage avec un équipement à presser les hamburgers, steaks, nuggets, cookies (avant cuisson) (patty pressing machine en anglais). Ainsi les demandes de brevets suivantes : US 2005/0226908 et les demandes de brevets de la même famille WO 2009/064859 et US 2009/0280159, WO 2012/049156, et US 2012/0141574 décrivent des tablettes à mâcher obtenues par moulage d'une pâte avec peu ou pas de pression (explicite dans les demandes WO 2009/064859 , US 2009/0280159, US 2012/0141574) préparée avec de l'eau (demande WO 2012/049156) ou présence d'eau (demande US 2005/0226908), utilisation de la chaleur (demandes US 2005/0226908, WO 2012/049156) toutes les demandes préconisant le même équipement de Formax Corporation : la Formax F6™. Compte tenu du mode de production à savoir l'utilisation d'un équipement à presser les hamburgers machine alimentée par une pâte bien que pouvant être homogène présente une densité aléatoire qui induit une quantité aléatoire dans le moule donc une quantité aléatoire de ou des substances actives pour être en particulier un médicament. Cela demande un parfait contrôle des caractéristiques physiques de la pâte. Malheureusement nous savons que nous ne sommes pas à l'abri d'une variation de la densité avec une pâte très peu fluide car moulée à froid. A cet inconvénient s'ajoute le système d'alimentation des moules qui aére la pâte, cela conduit inévitablement à une variation du poids des tablettes. Pour palier cela il faut peser une à une les tablettes à mâcher et écarter les hors normes qui ne pourront pas être recyclées sous peine de voir la mise à disposition de la substance active modifiée par une nouvelle mise en forme. Cette technique ne s'avère nullement économique. En conclusion elle ne sera pas retenue par l'Homme de l'Art pour faire un médicament ou un nutraceutique ou complément alimentaire où la quantité de substance(s) active(s) doit être parfaitement contrôlée.

La demanderesse s'est donné pour but de pallier les inconvénients de l'Art Antérieur et de mettre au point une composition et de trouver un procédé permettant de conférer aux compositions à administration orale pour les animaux sous forme solide une meilleure appétissance et une texture appréciée. Elle s'est en particulier employée à mettre au point une composition vétérinaire à administration orale simple adaptée et appréciée par chaque espèce animale, économique, dont la fabrication soit facilement industrialisable et que la quantité de chaque composant soit parfaitement contrôlée plus particulièrement pour le ou les substances actives s'agissant d'un médicament ou d'un nutraceutique ou complément alimentaire.

Dans les travaux qui ont conduit à la mise au point de la composition appétissante selon l'invention, la demanderesse a constaté que l'obtention de cette composition appétissante contenant le maximum de matières appétissantes par la technique de compression des bouillon cubes destinée en particulier à la compression des formules à forte teneur en matières grasses conduisait à des compositions vétérinaires à administration orale sous forme solide contenant une ou plusieurs substances actives parfaitement consommées par toutes les espèces animales. Cette technique permet de choisir une composition parfaitement adaptée donc appréciée par chacune des espèces animales cibles en choisissant bien les composants de la formule en cherchant à ce qu'ils soient dans leur majorité des matières appétissantes, la texture ainsi que la forme et la taille propres à l'espèce animale cible. Cette technique permet aussi de choisir les composants de la composition pour qu'ils ne soient pas la cause d'instabilité en particulier une dégradation du ou des substances actives. Cette technique permet de produire des compositions très homogènes et de garantir avec beaucoup de précision la quantité de substance(s) active(s) qu'elle contient. Cette technique est par ailleurs très économique et facilement industrialisable.

### Résumé de l'invention

L'invention se rapporte à 'une composition solide, son utilisation et son obtention, telle que définie par les revendications, comprenant au moins, par rapport au poids total de la composition :
- 5 à 30%, de préférence 8 à 20% en poids d'au moins une matière grasse, choisie parmi une huile liquide, un corps gras, une cire ou un mélange de ceux-ci, l'huile liquide ne pouvant représenter plus de 8% en poids de la composition,
- 0,001 à 55% en poids d'au moins une substance active, et
- 20 à 95%, de préférence 40 à 70% en poids d'au moins une matière appétissante,
- ladite composition étant obtenue par compression avec une presse à bouillon cubes d'un granulat homogène fluide présentant une granulométrie comprise entre 50 et 1000 µm, de préférence entre 200 et 600 µm,
- ladite technique de compression donnant des compositions présentant un poids unitaire très homogène ne s'écartant pas de plus ou moins de 3% de la valeur théorique du poids requis et préférentiellement de plus ou moins de 2% de la valeur théorique du poids requis,
- pour une utilisation en tant que médicament, nutraceutique ou complément alimentaire, à administration orale pour mammifère, excepté l'homme, en particulier pour l'animal domestique tel que le chien, le chat ou le cheval.

Par « substance active » on entend une substance médicamenteuse, nutraceutique ou complément alimentaire, ayant un effet thérapeutique ou ayant une activité biologique.

Dans les utilisations et compositions selon l'invention, l'huile liquide est choisie parmi l'huile d'olive, l'huile d'arachide, l'huile de colza, l'huile de tournesol, et leurs mélanges.

Dans les utilisations et compositions selon l'invention, le corps gras est choisi parmi la graisse de poulet, la graisse de canard, le saindoux, le suif, le beurre, la graisse de palme, la stéarine palmitique, la margarine, l'huile de palme éventuellement hydrogénée, l'huile de noix de coco hydrogénée, le palmitate de cétyle, et leurs mélanges.

Dans les utilisations et compositions selon l'invention, la cire est choisie parmi la cire d'abeille, la cire de carnauba, la cire de candelilla, et leurs mélanges.

Dans les utilisations et compositions selon l'invention, la matière appétissante est choisie parmi la viande, les farines de viande, les farines de poisson, les poudres de fromage, les dérivés du lait, la poudre de foie, la gélatine, les extraits de ces substances animales ou leurs dérivés ; la levure de bière ; les fibres végétales ; les produits ou les sous-produits de végétaux tels que le fenugrec, la pomme, la carotte, la betterave fourragère, la betterave sucrière, le thym, la luzerne, la canne à sucre, et les céréales tels que l'avoine, le blé, le riz et le maïs, le soja, leurs dérivés comme les farines, et leurs mélanges ; le sucre (saccharose) sous toutes ses formes, cristallisé, en poudre, le glucose, le sucre inverti, la mélasse, le miel ou ses dérivés ; et leurs mélanges, et le sel (chlorure de sodium).

On comprendra qu'une huile liquide, qu'un corps gras, et qu'une cire peuvent être aussi des matières appétissantes pour les animaux.

La composition comprend en outre un ou plusieurs additifs choisis de préférence soit dans le règne animal soit dans le règne végétal selon l'espèce animale cible parmi les charges, les liants, les solvants, les arômes, les tensioactifs, les exhausteurs de goût, les édulcorants, les anti-oxydants, les agents de chélation, les agents conservateurs, les colorants, et les régulateurs de pH.

De préférence, la charge est choisie parmi les maltodextrines ; les cyclodextrines ; le lactose ; le talc ; la silice ; les silicates ; les phosphates, la poudre de cellulose ; la cellulose microcristalline ; le mica et les carbonates.

De préférence, le liant est choisi parmi les polymères d'alcool polyvinylique, la polyvinylpyrrolidone, les copolymères de vinylpyrrolidone et d'acétate de vinyle, la carboxymethylcellulose, ses sels et ses dérivés, l'acide alginique et ses sels, la zéine, les pectines, la gomme arabique, la gomme d'acacia, la gomme adragante, la gomme karaya, la gomme xanthane, les carraghénanes, les polymères pullulan, les polymères agar, les amidons et leurs dérivés, les carbomères, l'acide acrylique réticulé avec des polyalkényle éthers, les polycarbophiles, et leurs mélanges.

De préférence, le solvant est choisi parmi l'éthanol, le propylène glycol, la glycérine, l'alcool cétylique, les polyéthylène glycols et leurs dérivés, et leurs mélanges.

De préférence, l'arôme est choisi parmi les huiles essentielles, les dérivés terpéniques tels que le menthol, et leurs mélanges.

De préférence, le tensioactifs est choisi parmi les esters de glycol comme le monostéarate de glycérol, les esters d'acides gras et de sorbitan, les esters d'acides gras polyoxyéthylénés et de sorbitan ; les huiles végétales polyoxyéthylénées comme les huiles de ricin polyoxyéthylénées, les huiles végétales hydrogénées polyoxyéthylénées comme les huiles de ricin hydrogénées polyoxyéthylénées ; la lécithine et ses dérivés de soja ou d'oeuf comme la phosphatidylcholine, la phosphatidylcholine hydrogénée, la lysophosphatidylcholine, la lysophosphatidylcholine hydrogénée, ainsi que leurs mélanges.

Dans un mode de réalisation préféré, l'exhausteur de goût est le glutamate de sodium ;

Dans un mode de réalisation préféré, l'édulcorant est choisi parmi l'aspartame ; le saccharinate de sodium ; la thaumatine; les polyols tels que le sorbitol, le xylitol, l'isomalt, le maltitol, le mannitol, et le lactitol ; et leurs mélanges ;

Préférentiellement, l'antioxydant est choisi parmi l'acide ascorbique, ses sels et ses dérivés, le métabisulfite de sodium ou potassium, le bisulfite de sodium, le butylhydroxyanisol, le butylhydroxytoluène, l'acide gallique et ses dérivés tel que le gallate de propyle, et leurs mélanges.

Préférentiellement, l'agent de chélation est choisi parmi l'EDTA et ses sels, l'acide tartrique et ses sels, et leurs mélanges.

Préférentiellement, l'agent conservateur est choisi parmi les parabens, l'acide benzoïque, le benzoate de sodium, l'acide sorbique, le sorbate de potassium, et leurs mélanges.

Préférentiellement, le colorant est choisi parmi les oxydes de fer, l'oxyde de titane, le curcumin, le caramel, les carotènes, et leurs mélanges.

Préférentiellement, le régulateur de pH est choisi parmi l'acide citrique, ses sels et ses dérivés, les carbonates sodiques, la delta glucono lactone, et leurs mélanges.

Dans un mode de réalisation préféré, le ou les additifs représente(nt) 0,01 à 75%, de préférence 1 à 50% en poids de la composition par rapport au poids total de la composition.

On comprendra qu'une charge peut avoir plusieurs fonctions ainsi un produit ou sous-produit de végétaux comme une farine de céréale ou le sucre peut être aussi bien une charge inerte qu'une matière appétissante.

De préférence, la substance active médicamenteuse est choisie parmi les anti-infectieux tels que les antibiotiques et les sulfamides, les cardiotoniques ; les antiparasitaires internes et externes ; les insecticides ; les inhibiteurs de croissance des insectes ; les anti-arthrosiques ; les anti-inflammatoires stéroïdiens ou non ; les antihistaminiques ; les hormones telles que les prostaglandines ; les substances de thérapie digestive tels que les pansements et sédatifs gastro-intestinaux, les antiulcéreux et les flores de substitution ; les anti-diarrhéiques ; les hépato-protecteurs ; les antispasmodiques ; les laxatifs ; les antiseptiques intestinaux ; les substances de thérapie respiratoire tels que les analeptiques respiratoires, les antitussifs, les broncho-dilatateurs, les fluidifiants bronchiques et mucolytiques, et les antiseptiques respiratoires ; les substances agissant sur le système nerveux tels que les analgésiques, les sédatifs et les tranquillisants ; les antiépileptiques ; les anesthésiques ; les orexigènes ; les anorexigènes , les substances de thérapie immunitaires telles que les interleukines et en particulier l'interféron ; les substances de thérapie anticancéreuses tels que les antimitotiques et les cytostatiques ; les macro-, micro- et oligo-éléments ; les vitamines ; les extraits de plantes ou d'organes d'animaux ; et leurs mélanges.

Dans un mode de réalisation avantageux, la substance active est choisie parmi les antibiotiques tels que l'amoxicilline, l'acide clavulanique, la céphalexine, la rifaximine ; les antiparasitaires tels que l'ivermectine, la moxidectine, la milbémycine le pyrantel, et ses dérivés tel que le pamoate , le praziquantel, les benzimidazoles , leurs sels ou leurs dérivés ; les insecticides tel que le fampronil ; les cardiotoniques tel que le levosimendan ; les anti-arthrosiques telle que la diaceréine.

De préférence, la substance active nutraceutique ou complément alimentaire est choisie parmi les extraits de plantes ou d'organes d'animaux pour leur action anti-infectieuse, antibactérienne, antifongique, anti-diarrhéique, hépato-protectrice, anti-spasmodique, laxative, anti-septique intestinal ; sur les problèmes respiratoires telle que la toux, en tant que broncho-dilatateurs, fluidifiant bronchique et mucolytique, antiseptique respiratoire, analgésique, sédatif, tranquillisant, anti-arthrosique, insecticide, antiparasitaire, anti-ulcère , anti-stress ; et les flores de substitution ; les macro-, micro- et oligo-éléments ; les vitamines ; et leurs mélanges.

Dans un mode de réalisation avantageux, la substance active nutraceutique ou complément alimentaire est choisie parmi les extraits de plantes ou d'animaux pour leur action anti-arthrosique tels que la chondroïtine sulfate, le chitosan et ses dérivés; pour leur action anti-ulcère et/ou anti-stress tel que l'extrait de soja fermenté ; pour leur action insecticide ou insectifuge tels que les pyrèthres ; les vitamines telles que la vitamine C, la vitamine D3 ; les flores de substitution tel que *Enterococcus faecium ;* les micro-éléments tel que le sélénium apporté par une souche de *Saccharomyces cerevisiae.*

On comprendra qu'une substance active peut avoir plusieurs fonctions ainsi l'extrait de soja fermenté peut être aussi bien une substance active qu'une matière appétissante.

### Description détaillée de l'invention

La description détaillée de l'invention qui est définie par les revendications sera faite ci-après notamment en s'appuyant sur des exemples donnés à titre de simple illustration et en référence aux dessins annexés sur lesquels :
- La figure 1 est une représentation schématique d'une presse à bouillon cubes, et
- la figure 2 illustre schématiquement un démotteur-calibreur avantageusement monté sur le réservoir d'alimentation de la presse à bouillons cubes.

La présente invention se rapporte à l'obtention et à l'utilisation de compositions appétissantes à administration orale sous forme solide parfaitement dosées, obtenues par compression, aux animaux. Les compositions ont une texture, une forme et une taille appréciées par les espèces animales. La présente invention se présente sous la forme d'un cube parfaitement dosé, appétissant très bien accepté par tous les animaux, quelle que soit l'espèce, absorbé rapidement, qu'il soit donné de façon occasionnelle ou répétée.

Les compositions de la présente invention sont obtenues par compression à l'aide d'une presse à bouillon cubes destinée en particulier à la compression de formules à forte teneur en gras.

Les compositions ou les « cubes à manger/mâcher » selon la présente invention sont obtenus par compression à l'aide d'une presse à bouillon cubes en bannissant tous les équipements travaillant en continu comme l'extrusion ou faisant intervenir une étape de fabrication en continu comme une extrusion-moulage, ou travaillant à partir de pâte ou de mélange hétérogène comme les équipements à presser les hamburgers, avec ou non présence de chaleur ou d'humidité.

Cette technique est largement utilisée dans le domaine alimentaire humain comme en témoignent les demandes de brevet suivantes: US 6,126,979, WO 2004/112513, WO 2006/063694, WO 2007/085609, WO 2009/068378, OA 12967 décrivant des compositions et l'utilisation de la compression pour former des bouillon cubes appelés aussi tablettes de bouillon. Leur emploi n'est pas direct, il faut au préalable les dissoudre dans de l'eau ou un véhicule aqueux et préférentiellement chaud ou au moins tiède. De plus ces bouillon cubes sont très friables car ils doivent pouvoir être émiettés facilement sur les plats à cuisiner. De ce fait l'Homme de l'Art ne se tournerait pas vers ces formulations et leur procédé de réalisation pour obtenir une composition solide appétissante ayant la texture souhaitée.

Les compositions solides appétissantes obtenues sont utilisées pour être administrées aux animaux pour un traitement thérapeutique soit médicamenteux soit nutraceutique ou complément alimentaire, les animaux étant plus particulièrement des mammifères.

Les compositions selon la présente invention se présentent typiquement sous la forme de cubes à manger/mâcher utilisés pour faciliter la prise orale par les animaux, de préférence les mammifères.

Même si la composition appétissante à administration orale sous forme solide pour les animaux est désignée comme "cube à manger/mâcher",aussi d'autres formes en respectant les contraintes de l'équipement à savoir qu'au moins les deux faces supérieure et inférieure du cube à manger/mâcher sont planes.

Le procédé de compression des bouillon cubes est connu de l'art antérieur, et est notamment utilisé dans le domaine de l'alimentation humaine. Ces produits sont typiquement les bouillon cubes. Cependant les compositions, telles que les bouillon cubes, obtenues par ce procédé de compression, n'ont jusqu'à présent jamais été utilisées dans le domaine vétérinaire c'est-à-dire appliqués données directement aux animaux, plus particulièrement aux mammifères par prise directe par voie orale à l'exception de l'homme qui les consomme indirectement soit dissous dans de l'eau chaude ou tiède soit émiettés sur ses plats.

Par rapport à d'autres techniques de fabrication de produits la voie orale pour l'animal décrites dans l'art antérieur dont notamment la compression de poudre sèche, l'extrusion, et du moulage, la compression des bouillon cubes permet d'obtenir des produits plus riches en matière grasse et en matière appétissante ce qui conduit à une parfaite appétence c'est-à-dire à une prise totale même si elle est répétitive par l'animal. Toutes les compositions contiennent une substance active, ce procédé de compression permet de contrôler parfaitement le poids du cube à manger/mâcher donc la quantité de substance active présente dans chaque unité du produit final et garantir ainsi le traitement thérapeutique.

Les procédés de l'art antérieur,
- la compression de poudre sèche ne permet pas de fabriquer des produits suffisamment riches en matières appétissantes, ayant une texture appréciés des animaux,
- les procédés d'extrusion et d'extrusion-moulage ne permettent pas de fabriquer des produits suffisamment riches en gras n'exudant pas, tout en dosant parfaitement le produit.
- les procédés de moulage de hamburgers ne permettent pas de fabriquer des produits suffisamment riches en gras n'exudant pas, tout en dosant parfaitement le produit.

Ainsi, les produits fabriqués notamment par extrusion ou le moulage présentent en général de fortes variations de poids. Ces variations sont dues à différents facteurs comme l'alimentation de l'équipement en mélange, mais aussi au mélange lui-même sous forme de pâte se formant dans les vis de l'extrudeur ou directement pour le moulage, pâte qui présente une forte hétérogénéité. Ces variations peuvent être compensées par un tri des produits en sortie des chaînes de fabrication mais un tel tri est associé à une perte importante de produit entraînant un surcoût non négligeable. Le calibrage des produits est particulièrement déterminant en ce qui concerne les compositions incorporant une substance active. Pour permettre l'administration d'une quantité constante de substance active, le produit final doit être parfaitement calibré quant à son poids. Ce calibrage est obtenu avantageusement en fabriquant les compositions selon la présente invention par compression des bouillons cubes.

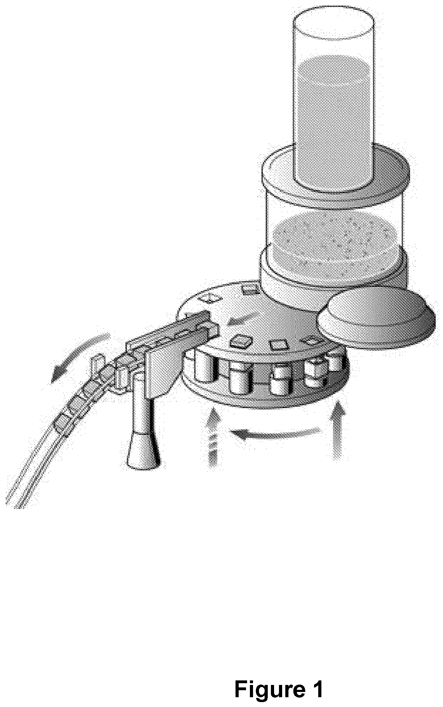

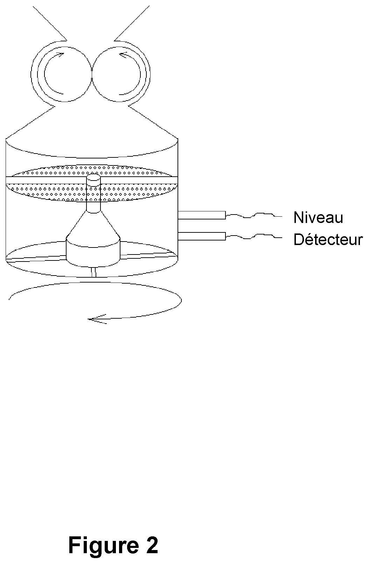

L'invention se rapporte donc à un procédé de fabrication d'un cube à manger/mâcher pour animaux comprenant les étapes suivantes :
- introduction des composants pulvérulents, 0,001 à 55% en poids d'au moins une substance active, et 20 à 95%, de préférence 40 à 70% en poids d'au moins une matière appétissante, éventuellement un ou plusieurs additifs en ayant soin de préférer des composants avec une granulométrie fine, inférieure à 200 µm et de préférence inférieure à 100 µm, dans un mélangeur-granulateur vertical,
- mélange sans apport de chaleur ou d'eau,
- introduction des composants liquides et éventuellement d'un ou plusieurs additifs liquides, des composants dissous dans une matière grasse liquide ou un additif liquide,
- introduction des matières grasses, mélange de 5 à 30%, de préférence 8 à 20% en poids d'au moins une matière grasse, choisie parmi une huile liquide, un corps gras, une cire ou un mélange de ceux-ci, l'huile liquide ne pouvant représenter plus de 8% en poids du cube à manger/mâcher :
   - par vaporisation des matières grasses liquides à température ambiante, ou
   - par vaporisation des matières grasses se présentant en masse préalablement liquéfiées, ou
   - directement pour les matières grasses en poudre présentant une granulométrie fine, inférieure à 200 µm et de préférence inférieure à 100 µm,
   sous agitation jusqu'à l'obtention d'un granulat homogène sec, fluide, ne présentant pas d'agglomérats, de prise en masse,
- calibrage à 600 µm du granulat,
- compression du granulat sec, fluide et calibré, avec un équipement standard comme les presses à bouillon cubes de Fette ou Bonals, (figure 1 : schéma du principe de fonctionnement de la presse), donnant des masses compactes et homogènes.
L'équipement de fabrication pour la mise en oeuvre du procédé ci-dessus est avantageusement modifié :
- par l'ajout d'un démotteur-calibreur sur le réservoir d'alimentation de la presse à bouillon cubes qui permet d'améliorer le remplissage de l'alvéole par un volume constant c'est-à-dire faire le volume de granulat donc le poids introduit dans l'alvéole soit le plus constant possible afin de garantir une très faible variation du poids du cube à manger/mâcher résultant par rapport au poids théorique fixé, en conséquence de quoi la quantité de substance active présente dans le cube sera parfaitement constante (figure 2: démotteur-calibreur), et
- pour améliorer l'extraction du cube à manger/mâcher, mais aussi éviter tout échauffement qui pourrait être préjudiciable à la stabilité cube/substance active, l'étage de compression est réfrigéré.

La dureté des cubes à manger/mâcher peut être contrôlée par ajustement de la pression exercée par la presse à bouillon cubes et par la composition.

Ladite technique de compression donne des objets présentant un poids unitaire très homogène ne s'écartant pas de plus ou moins de 3% de la valeur théorique du poids requis et préférentiellement de plus ou moins de 2% de la valeur théorique du poids requis, ce qui permet une utilisation :
- comme médicament pour mammifère, excepté l'homme, en particulier pour animal domestique tel que le chien, le chat ou le cheval, ou
- comme nutraceutique ou complément alimentaire pour mammifère, excepté l'homme, en particulier pour animal domestique tel que le chien, le chat ou le cheval.

Par « huile », on entend une matière grasse qui est liquide à la température ambiante. En général, l'huile est d'origine végétale, bien qu'elle puisse être d'origine animale ou minérale. Selon l'espèce cible la préférence ira aux huiles d'origine végétale ou animale.

Selon l'invention, le cube à manger/mâcher peut contenir une huile liquide acceptable n'ayant aucune activité dégradante vis-à-vis des autres composants choisis en particulier la substance active.

Une meilleure diffusion de l'huile liquide à travers la masse des composants de la formule facilite l'homogénéité et améliore l'aspect du cube à manger/mâcher et cela est d'autant plus vrai que la graisse est remplacée au moins partiellement par de l'huile liquide.

Dans les utilisations et compositions selon l'invention, l'huile liquide est de préférence choisie parmi l'huile d'olive, l'huile d'arachide, l'huile de colza, l'huile de tournesol, et leurs mélanges.

Par « corps gras », on entend une matière contenant un ou plusieurs lipides.

Dans les utilisations et compositions selon l'invention, le corps gras est de préférence choisi soit dans le règne animal soit dans le règne végétal selon l'espèce animale cible parmi les graisses pâteuses ou dures, et est choisi parmi la graisse de poulet, la graisse de canard, le saindoux, le suif, le beurre, la graisse de palme, la stéarine palmitique, la margarine, l'huile de palme éventuellement hydrogénée, le palmitate de cétyle, l'huile de noix de coco hydrogénée et leurs mélanges.

Par « cires » on entend les cires autorisées dans l'alimentation et notamment dans l'alimentation animale.

Dans les utilisations et compositions selon l'invention, la cire est de préférence choisie soit dans le règne animal soit dans le règne végétal selon l'espèce animale cible. Elle est choisie parmi la cire d'abeille, la cire de carnauba, la cire de candelilla, et leurs mélanges.

Dans les utilisations et compositions selon l'invention, les huiles liquides, les corps gras ou les cires vont permettre une granulation du mélange homogène des composants pulvérulents.

On comprendra qu'une huile liquide, qu'un corps gras, et qu'une cire peuvent être aussi des matières appétissantes pour les animaux.

Les compositions selon la présente invention comprennent au moins une matière appétissante en forte quantité contribuant aux caractères organoleptiques de la composition selon l'invention et son appétence pour les animaux.

Les matières appétissantes pour l'animal cible sont par exemple les substances d'origine animale ou végétale, directement mises en poudre après traitements tels que le séchage ou la déshydratation, le broyage, le calibrage mais aussi après transformation avec ajout d'autres composants pour favoriser la conservation par exemple. Les composants appétants sont choisis parmi les substances de choix qui ont une forte appétibilité pour les espèces cibles, en particulier les carnivores domestiques tels que les chiens et les chats ou les herbivores comme le cheval.

Dans les utilisations et compositions selon l'invention, la matière appétissante est choisie soit dans le règne animal soit dans le règne végétal selon l'espèce animale cible de préférence. Elle est choisie parmi la viande, les farines de viande, les farines de poisson, les poudres de fromage, les dérivés du lait, la poudre de foie, la gélatine, les extraits de ces substances animales ou leurs dérivés ; la levure de bière ; les fibres végétales ; les produits ou les sous-produits de végétaux tels que le fenugrec, la pomme, la carotte, la betterave fourragère, la betterave sucrière, le thym, la luzerne, la canne à sucre, et les céréales tels que l'avoine, le blé, le riz et le maïs, le soja, leurs dérivés comme les farines, et leurs mélanges ; le sucre (saccharose) sous toutes ses formes, cristallisé, en poudre, le glucose, le sucre inverti, la mélasse, le miel ou ses dérivés ; et leurs mélanges ; soit le sel (chlorure de sodium).

La composition comprend en outre un ou plusieurs additifs choisis de préférence soit dans le règne animal soit dans le règne végétal selon l'espèce animale cible parmi les charges, les liants, les solvants, les arômes, les tensioactifs, les exhausteurs de goût, les édulcorants, les anti-oxydants, les agents de chélation, les agents conservateurs, les colorants, et les régulateurs de pH.

De préférence, la charge est choisie parmi les maltodextrines ; les cyclodextrines ; le lactose ; le talc ; la silice ; les silicates ; les phosphates, la poudre de cellulose ; la cellulose microcristalline ; le mica et les carbonates.

De préférence, le liant est choisi parmi les polymères d'alcool polyvinylique, la polyvinylpyrrolidone, les copolymères de vinylpyrrolidone et d'acétate de vinyle, la carboxymethylcellulose, ses sels et ses dérivés, l'acide alginique et ses sels, la zéine, les pectines, la gomme arabique, la gomme d'acacia, la gomme adragante, la gomme karaya, la gomme xanthane, les carraghénanes, les polymères pullulan, les polymères agar, les amidons et leurs dérivés, les carbomères, l'acide acrylique réticulé avec des polyalkényle éthers, les polycarbophiles, et leurs mélanges.

De préférence, le solvant est choisi parmi l'éthanol, le propylène glycol, la glycérine, l'alcool cétylique, les polyéthylène glycols et leurs dérivés, et leurs mélanges.

Un arôme est un principe odorant de certaine substance (d'origine synthétique ou naturelle) qui ne sera perçu que par l'odorat. Il ne produira pas de sensation sur l'organe du goût et n'aura donc pas de saveur.

De préférence, l'arôme est choisi parmi les huiles essentielles, les dérivés terpéniques tels que le menthol, et leurs mélanges.

De préférence, le tensioactif est choisi parmi les esters de glycol comme le monostéarate de glycérol, les esters d'acides gras et de sorbitan, les esters d'acides gras polyoxyéthylénés et de sorbitan ; les huiles végétales polyoxyéthylénées comme les huiles de ricin polyoxyéthylénées, les huiles végétales hydrogénées polyoxyéthylénées comme les huile s de ricin hydrogénées polyoxyéthylénées ; la lécithine et ses dérivés de soja ou d'oeuf comme la phosphatidylcholine, la phosphatidylcholine hydrogénée, la lysophosphatidylcholine, la lysophosphatidylcholine hydrogénée, ainsi que leurs mélanges.

Dans un mode de réalisation préféré, l'exhausteur de goût est le glutamate de sodium .

Dans un mode de réalisation préféré, l'édulcorant est choisi parmi l'aspartame ; le saccharinate de sodium ; la thaumatine; les polyols tels que le sorbitol, le xylitol, l'isomalt, le maltitol, le mannitol, et le lactitol ; et leurs mélanges.

Préférentiellement, l'antioxydant est choisi parmi l'acide ascorbique, ses sels et ses dérivés, le métabisulfite de sodium ou potassium, le bisulfite de sodium, le butylhydroxyanisol, le butylhydroxytoluène, l'acide gallique et ses dérivés tels que le gallate de propyle, et leurs mélanges.

Préférentiellement, l'agent de chélation est choisi parmi l'EDTA et ses sels, l'acide tartrique et ses sels, et leurs mélanges.

Préférentiellement, l'agent conservateur est choisi parmi les parabens, l'acide benzoïque, le benzoate de sodium, l'acide sorbique, le sorbate de potassium, et leurs mélanges.

Préférentiellement, le colorant est choisi parmi les oxydes de fer, l'oxyde de titane, le curcumin, le caramel, les carotènes, et leurs mélanges.

Préférentiellement, le régulateur de pH est choisi parmi l'acide citrique, ses sels et ses dérivés, les carbonates sodiques, la delta glucono lactone, et leurs mélanges.

Dans un mode de réalisation préféré, le ou les additif(s) représente(nt) 0,01 à 75%, de préférence 1 à 50% en poids de la composition par rapport au poids total de la composition.

On comprendra qu'une charge peut avoir plusieurs fonctions ainsi un produit ou sous-produit de végétaux comme une farine de céréale ou le sucre peut être aussi bien une charge inerte qu'une matière appétissante.

Par « substance active » on entend la substance d'un médicament, d'un nutraceutique ou d'un complément alimentaire ayant un effet thérapeutique ou ayant une activité biologique.

La ou les substances actives peuvent être simplement réparties au sein du cube à manger ou être préalablement encapsulées ou enrobées par les techniques connues de l'homme du métier afin d'améliorer leur stabilité ou d'augmenter le masquage de leur odeur et de leur goût à la perception olfactive ou gustative de l'animal ou réparties par vaporisation d'une solution non aqueuse dans un solvant ou mieux dans l'huile de la composition suivant la ou leur stabilité dans le solvant.

De préférence, la substance active médicamenteuse est choisie parmi les anti-infectieux tels que les antibiotiques et les sulfamides, les cardiotoniques ; les antiparasitaires internes et externes ; les insecticides ; les inhibiteurs de croissance des insectes ; les anti-arthrosiques ; les anti-inflammatoires stéroïdiens ou non ; les antihistaminiques ; les hormones telles que les prostaglandines ; les substances de thérapie digestive tels que les pansements et sédatifs gastro-intestinaux, les antiulcéreux et les flores de substitution ; les anti-diarrhéiques ; les hépato-protecteurs ; les antispasmodiques ; les laxatifs ; les antiseptiques intestinaux ; les substances de thérapie respiratoire tels que les analeptiques respiratoires, les antitussifs, les broncho-dilatateurs, les fluidifiants bronchiques et mucolytiques, et les antiseptiques respiratoires ; les substances agissant sur le système nerveux tels que les analgésiques, les sédatifs et les tranquillisants; les antiépileptiques ; les anesthésiques ; les orexigènes ; les anorexigènes , les substances de thérapie immunitaires telles que les interleukines et en particulier l'interféron ; les substances de thérapie anticancéreuses tels que les antimitotiques et les cytostatiques ; les macro-, micro- et oligo-éléments ; les vitamines ; les extraits de plantes ou d'organes d'animaux ; et leurs mélanges.

Dans un mode de réalisation avantageux, la substance active est choisie parmi les antibiotiques tels que l'amoxicilline, l'acide clavulanique, la céphalexine, la rifaximine ; les antiparasitaires tels que l'ivermectine, la moxidectine, la milbémycine le pyrantel et ses dérivés tel que le pamoate , le praziquantel, les benzimidazoles , leurs sels ou leurs dérivés ; les insecticides tel que le fampronil ; les cardiotoniques tel que le levosimendan ; les anti-arthrosiques telle que la diaceréine.

De préférence, la substance active nutraceutique ou complément alimentaire est choisie parmi les extraits de plantes ou d'animaux pour leur action anti-infectieuse, antibactérienne, antifongique, anti-diarrhéique, hépato-protectrice, anti-spasmodique, laxative, anti-septique intestinal ; sur les problèmes respiratoires telle que la toux, en tant que broncho-dilatateurs, fluidifiant bronchique et mucolytique, antiseptique respiratoire, analgésique, sédatif, tranquillisant, anti-arthrosique, insecticide, antiparasitaire, anti-ulcère , anti-stress ; et les flores de substitution ; les macro-, micro- et oligo-éléments ; les vitamines ; et leurs mélanges.

Dans un mode de réalisation avantageux, la substance active nutraceutique ou complément alimentaire est choisie parmi les extraits de plantes ou d'organes d'animaux pour leur action anti-arthrosique tels que l'acide chondroïtique sulfate, le chitosan et ses dérivés; pour leur action anti-ulcère et/ou anti-stress tel que l'extrait de soja fermenté ; pour leur action insecticide ou insectifuge tels que les pyrèthres ; les vitamines telles que la vitamine C, la vitamine D3 ; les flores de substitution tel que *Enterococcus faecium ;* les micro-éléments tel que le sélénium apporté par une souche de *Saccharomyces cerevisiae.*

On comprendra qu'une substance active peut avoir plusieurs fonctions ainsi l'extrait de soja fermenté peut être aussi bien une substance active qu'une matière appétissante.

Selon un mode de réalisation préféré, l'invention concerne une :
i) Composition appétissante solide comprenant par rapport au poids total de la composition :
   - 5 à 30%, de préférence 8 à 20% en poids d'au moins une matière grasse, choisie parmi une huile liquide, un corps gras, une cire ou un mélange de ceux-ci, l'huile liquide ne pouvant représenter plus de 8% en poids de la composition,
   - 0,001 à 55% en poids d'au moins une substance active, et
   - 20 à 95%, de préférence 40 à 70% en poids d'au moins une matière appétissante,
   pour son utilisation en tant que médicament à administration orale aux mammifères, excepté l'homme, en particulier pour l'animal domestique tel que le chien, le chat ou le cheval ;
   ou
ii) Composition appétissante solide comprenant, par rapport au poids total de la composition :
   - 5 à 30%, de préférence 8 à 20% en poids d'au moins une matière grasse, choisie parmi une huile liquide, un corps gras, une cire ou un mélange de ceux-ci, l'huile liquide ne pouvant représenter plus de 8% en poids de la composition,
   - 0,001 à 55% en poids d'au moins une substance active, et
   - 20 à 95%, de préférence 40 à 70% en poids d'au moins une matière appétissante,
   pour son utilisation en tant que nutraceutique ou complément alimentaire à administration orale aux mammifères, excepté l'homme, en particulier pour l'animal domestique tel que le chien, le chat ou le cheval ,
   caractérisée en ce que :
   a) l'huile liquide est choisie parmi l'huile d'olive, l'huile d'arachide, l'huile de colza, l'huile de tournesol, et leurs mélanges ;
   b) le corps gras est choisi parmi la graisse de poulet, la graisse de canard, le saindoux, le suif, le beurre, la graisse de palme, la stéarine palmitique, la margarine, l'huile de palme éventuellement hydrogénée, le palmitate de cétyle, l'huile de noix de coco hydrogénée et leurs mélanges ;
   c) la cire est choisie parmi la cire d'abeille, la cire de carnauba, la cire de candelilla, et leurs mélanges ;
   d) la matière appétissante est choisie parmi la viande, les farines de viande, les farines de poisson, les poudres de fromage, les dérivés du lait, la poudre de foie, la gélatine, les extraits de ces substances animales ou leurs dérivés ; la levure de bière ; les fibres végétales ; les produits ou les sous-produits de végétaux tels que le fenugrec, la pomme, la carotte, la betterave fourragère, la betterave sucrière, le thym, la luzerne, la canne à sucre, les céréales tels que l'avoine, le blé, le riz et le maïs, le soja, leurs dérivés comme les farines, et leurs mélanges ; le sucre cristallisé, en poudre (saccharose), le glucose, le sucre inverti, la mélasse, le miel et ses dérivés, et leurs mélanges, le sel (chlorure de sodium), et
   e) ladite composition solide est obtenue par mélange des composants, vaporisation des matières grasses, calibrage du granulat sec et fluide, et compression du granulat avec une presse à bouillon cubes, où
      - le mélange se présente sous forme d'un granulat homogène, sec, fluide,
      - le granulat sec et fluide présente une granulométrie comprise entre 50 et 1000 µm, de préférence entre 200 et 600 µm,
      - la composition appétissante solide présente un poids unitaire de plus ou moins de 3% de la valeur théorique du poids requis, et préférentiellement de plus ou moins de 2% de la valeur théorique du poids requis.

Les exemples qui suivent sont donnés uniquement à titre d'illustration de l'objet de l'invention dont ils ne constituent en aucune manière une limitation.

### Exemples

### EXEMPLE 1 - Préparation de cube à manger/mâcher pour chien selon l'invention

Des cubes à manger/mâcher- contenant 68 µg d'ivermectine, et 57,5 mg de pyrantel sous forme de pamoate selon l'invention sont réalisés avec la composition suivante :

| | |
|---|---|
| Ivermectine | 0,001236 % |
| Pamoate de pyrantel | 2,963636% |
| Huile de colza | 5,00 % |
| Suif | 10,00 % |
| Viande de boeuf | 40,30% |
| Farine de soja | 28,132428 % |
| Sel | 2,00 % |
| Mélasse | 8,00 % |
| Glucono delta lactone | 3,30 % |
| Sorbate de potassium | 0,30 % |
| Propyl gallate | 0,0005 % |
| BHA | 0,0018 % |
| Acide citrique | 0,0004 % |

L'ivermectine est introduite sous fome de prémélange obtenu à partir d'ivermectine, huile de colza, et des antioxydants.

Dans un mélangeur introduire la viande de boeuf, la farine de soja, le sorbate de potassium, le sel, la glucono delta lactone, l'acide citrique, et le pamoate de pyrantel, et mélanger.

Verser sur le mélange pulvérulent homogène le prémélange d'ivermectine, le suif liquéfié, et la mélasse. Mélanger jusqu'à l'obtention d'un granulat homogène, sec et fluide.

Calibrer le granulat sur un tamis de 600 µm.

Les cubes à manger/mâcher_de forme parallélépipédique de dimension 13,95 x 13,95 x 25,0 mm sont obtenus par compression à l'aide d'une presse à bouillon cubes.

Tous les cubes à manger ont été pesés séparément, leur poids théorique est de 5,5 g.

Aucun cube ne s'écartait de plus de 2 pour cent de la masse théorique. Cela n'aurait pas été réalisable en utilisant les techniques habituellement mise en oeuvre à savoir l'extrusion qui est une fabrication en continu ou le moulage utilisant une pâte.

La détermination de la teneur en Ivermectine pour les 5 cubes à manger/mâcher les plus légers et les 5 plus lourd a montré que le mélange était parfaitement homogène car les valeurs de dosage se trouvaient toutes dans l'intervalle de doses, admis pour les médicaments, encadrant la dose théorique.

### EXEMPLE 2 - test monadique d'appétence avec les cubes à manger/mâcher

Un test d'appétence des cubes à manger/mâcher obtenus dans l'exemple 1 a été réalisé auprès de trente chiens adultes, mâles et femelles, de races variées en test croisé.

Les chiens de petites tailles jusqu'à 11 kg reçoivent 1 cube, les chiens de taille moyenne de 11 à 22 kg reçoivent 2 cubes, et les chiens de grande taille reçoivent 3 cubes. Quand il y a plusieurs cubes, ils sont proposés en même temps.

Il s'agit d'un test monadique réalisé dans des box individuels pendant dix minutes par chiens. Ont été mesurés :
- la préhension
   ✔ à la main,
   ✔ au sol, ou
   ✔ pas de prise
- la consommation
   ✔ totale,
   ✔ partielle, ou
   ✔ pas de consommation.

Pour chacun de ces critères est précisé le nombre d'individus, sur l'ensemble du panel et par catégories de taille (petite/moyenne/grande).
Le calcul de l'acceptabilité est basé sur le pourcentage de chiens ayant consommé la totalité du cube à manger/mâcher.

### Préhension :

| | petits chiens | chiens moyens | grands chiens |
|---|---|---|---|
| A la main | 10 | 10 | 10 |
| au sol | 0 | 0 | 0 |
| pas de prise | 0 | 0 | 0 |

### Consommation :

| | petits chiens | chiens moyens | grands chiens |
|---|---|---|---|
| partielle | 0 | 0 | 0 |
| totale | 10 | 10 | 10 |
| pas de consommation | 0 | 0 | 0 |

L'acceptabilité et la consommation des cubes à manger/mâcher selon l'invention sont totales (100%).

### EXEMPLE 3 - test comparatif d'homogénéité des poids entre les cubes à manger/mâcher et des tablettes à mâcher obtenues par extrusion

Vingt cubes à manger/mâcher, obtenus selon l'invention conforme à l'exemple 1, pris consécutivement en sortie de machine ont été pesés.

Un lot de tablettes à mâcher a été réalisé par extrusion avec la formule suivante :

| | |
|---|---|
| Ivermectine | 0,001236 % |
| Pamoate de pyrantel | 2,963636% |
| Suif | 2,5 % |
| Viande de boeuf | 42,0 % |
| Farine de soja | 30,932404 % |
| Sel | 2,00 % |
| Mélasse | 8,00 % |
| Propylène glycol | 5,0 % |
| Glucono delta lactone | 3,30 % |
| Sorbate de potassium | 0,30 % |
| Propyl gallate | 0,0005 % |
| BHA | 0,0018 % |
| Acide citrique | 0,0004 % |

De même vingt tablettes, prises consécutivement en sortie d'extrusion ont été pesées.

Les poids relevés pour les deux prélèvements des vingt objets sont donnés dans le tableau suivant :

| N° de l'objet | Cube à mâcher Poids en grammes | Tablette à mâcher Poids en grammes |
|---|---|---|
| 1 | 5.53 | 5.55 |
| 2 | 5.56 | 4.83 |
| 3 | 5.51 | 5.92 |
| 4 | 5.49 | 5.49 |
| 5 | 5.42 | 6.24 |
| 6 | 5.41 | 4.86 |
| 7 | 5.53 | 5.69 |
| 8 | 5.56 | 6.27 |
| 9 | 5.60 | 4.78 |
| 10 | 5.59 | 4.96 |
| 11 | 5.55 | 5.67 |
| 12 | 5.48 | 6.13 |
| 13 | 5.45 | 6.06 |
| 14 | 5.49 | 5.19 |
| 15 | 5.50 | 5.43 |
| 16 | 5.47 | 4.99 |
| 17 | 5.57 | 4.75 |
| 18 | 5.52 | 5.78 |
| 19 | 5.49 | 6.01 |
| 20 | 5.55 | 5.26 |
| Ecart | < 2% | > 14% |

Un écart important, supérieur à 14%, est observé entre les poids extrêmes des tablettes à mâcher et le poids moyen obtenu avec les 20 tablettes à mâcher obtenues par extrusion comparativement à l'écart de poids, inférieur à 2%, observé pour les cubes à manger/mâcher obtenus selon l'invention.

Ces écarts de poids entraînent obligatoirement une variation d'au moins la même importance des quantités de molécules actives contenues dans les tablettes à mâcher. Il est inenvisageable d'utiliser le procédé d'extrusion ou de moulage pour la réalisation de médicaments à prise unitaire, si ce n'est en pesant les tablettes à mâcher une par une en sortie du conformateur, et d'écarter toutes celles qui ne rentraient pas dans le domaine de poids garantissant une quantité de molécules actives telle que l'impose la réglementation des médicaments. Si en première approche, dans l'exemple présenté, n'étaient conservées que les tablettes à mâcher dont le poids ne s'écarte pas de plus de 5 pour cent du poids théorique, il ne serait gardé que 6 tablettes à mâcher sur les vingt produites, soit 30 pour cent de la production ce qui n'est pas économique et sans garantie de la quantité de molécules actives pour les tablettes à mâcher dont le poids est proche ou équivalent aux poids limites supérieure et inférieure pour sélectionner les tablettes à mâcher. De même il est inenvisageable d'extruder ou de mouler une deuxième fois les tablettes à mâcher écartées car inévitablement la mise à disposition des molécules actives dans l'organisme serait modifiée et entraînerait une modification de l'activité thérapeutique.

### EXEMPLE 4 - Préparation de cubes à manger/mâcher pour chevaux selon l'invention

Des cubes à manger/mâcher, sont réalisés avec la composition suivante :

| | |
|---|---|
| Extrait de soja fermenté | 83 % |
| Huile de palme hydrogénée | 10 % |
| Sucre cristallisé | 6 % |
| Oxyde de titane | 0,9 % |
| BHA | 0,1 % |

Mélange du sucre cristallisé, de l'extrait de soja fermenté, du BHA et de l'oxyde de titane, et pulvérisation sur le mélange de l'huile de palme hydrogénée préalablement liquéfié. Calibrage du granulat obtenu sur un tamis de 600 µm.

Les cubes à manger/mâcher de forme parallélépipédique de dimension 31 x 23 x 10 mm sont obtenus par compression à l'aide d'une presse à bouillon cubes.

Tous les cubes à manger/mâcher ont été pesés séparément. Aucun cube ne s'écartait de plus de 2 pour cent de la masse théorique.

### EXEMPLE 5 - test d'appétence avec les cubes à manger/mâcher de l'exemple 4

Un test d'appétence des cubes à manger/mâcher obtenus dans l'exemple 4 a été réalisé auprès de plusieurs propriétaires de chevaux et poneys et dans un club hippique.

Le cube à manger/mâcher a été présenté au cheval ou au poney sur la main grande ouverte.

L'acceptabilité et la consommation des cubes à manger ont été totale (100 %).

Il y a lieu de mentionner le comportement des animaux vis-à-vis de ce cube. Plusieurs cavaliers ont rapporté la grande attirance qu'avaient les chevaux et les poneys pour le cube à manger/mâcher présenté sous cette forme taille et couleur. Dès le premier absorbé, ils cherchaient à en avoir d'autres en sentant les poches du cavalier d'où étaient sortis ces cubes.

### EXEMPLE 6 - Préparation de cube à manger/mâcher pour chien selon l'invention

Des cubes à manger/mâcher de 3g contenant 68 µg d'ivermectine, 57 mg de pyrantel sous forme de pamoate, et 57 mg de praziquantel selon l'invention sont réalisés avec la composition suivante :

| | |
|---|---|
| Ivermectine enrobée | 1,0 % |
| Pamoate de pyrantel enrobé | 6,0 % |
| Praziquantel enrobé | 5,0 % |
| Poudre de foie | 46,00 % |
| Graisse dure | 8,00 % |
| Glycérine | 12,00 % |
| PEG 4000 | 12,00 % |
| Gélatine | 6,00 % |
| Sucre | 3,54 % |
| Acide sorbique | 0, 40 % |
| BHA | 0, 04 % |
| Propyl gallate | 0, 02 % |

Dans un mélangeur introduire la poudre de foie, le PEG 4000, la gélatine, le sucre, l'acide sorbique, le BHA, le propyl gallate, l'ivermectine granulée, le pamoate de pyrantel granulé, et le praziquantel granulé, et mélanger.

Verser sur le mélange pulvérulent homogène la graisse dure liquéfiée, et la glycérine. Mélanger jusqu'à l'obtention d'un granulat homogène, sec et fluide.

Calibrer le granulat sur un tamis de 600 µm.

Les cubes à manger/mâcher de forme parallélépipédique de dimension 17 x 17 x 9,6 mm sont obtenus par compression à l'aide d'une presse à bouillon cubes.

Tous les cubes à manger/mâcher ont été pesés séparément, leur poids théorique est de 3,0 g.

Aucun cube ne s'écartait de plus de 2 pour cent de la masse théorique.

## Revendications

1. Composition appétissante solide du type médicament, nutraceutique ou complément alimentaire, à administration orale aux mammifères, excepté l'homme, en particulier pour l'animal domestique tel que le chien, le chat ou le cheval comprenant par rapport au poids total de la composition :
- 5 à 30%, de préférence 8 à 20% en poids d'au moins une matière grasse, choisie parmi une huile liquide, un corps gras, une cire ou un mélange de ceux-ci, l'huile liquide ne pouvant représenter plus de 8% en poids de la composition,
- 0,001 à 55% en poids d'au moins une substance active, et
- 20 à 95%, de préférence 40 à 70% en poids d'au moins une matière appétissante
**caractérisée en ce que** :
a) l'huile liquide est choisie parmi l'huile d'olive, l'huile d'arachide, l'huile de colza, l'huile de tournesol, et leurs mélanges ;
b) le corps gras est choisi parmi la graisse de poulet, la graisse de canard, le saindoux, le suif, le beurre, la graisse de palme, la stéarine palmitique, la margarine, l'huile de palme éventuellement hydrogénée, le palmitate de cétyle, l'huile de noix de coco hydrogénée et leurs mélanges ;
c) la cire est choisie parmi la cire d'abeille, la cire de carnauba, la cire de candelilla, et leurs mélanges ;
d) la matière appétissante est choisie parmi la viande, les farines de viande, les farines de poisson, les poudres de fromage, les dérivés du lait, la poudre de foie, la gélatine, les extraits de ces substances animales ou leurs dérivés ; la levure de bière ; les fibres végétales ; les produits ou les sous-produits de végétaux tels que le fenugrec, la pomme, la carotte, la betterave fourragère, la betterave sucrière, le thym, la luzerne, la canne à sucre, les céréales tels que l'avoine, le blé, le riz et le maïs, le soja, leurs dérivés comme les farines, et leurs mélanges ; le saccharose cristallisé, en poudre, le glucose, le sucre inverti, la mélasse, le miel et ses dérivés, et leurs mélanges, le chlorure de sodium, et
e) ladite composition solide est obtenue par mélange des composants, vaporisation des matières grasses, calibrage du granulat sec et fluide, et compression du granulat avec une presse à bouillon cubes, où
- le mélange se présente sous forme d'un granulat homogène, sec, fluide,
- le granulat sec et fluide présente une granulométrie comprise entre 50 et 1000 µm, de préférence entre 200 et 600 µm,
- la composition appétissante solide présente un poids unitaire de plus ou moins de 3% de la valeur théorique du poids requis, et préférentiellement de plus ou moins de 2% de la valeur théorique du poids requis.

2. Composition selon la revendication 1 **caractérisée en ce que** la composition comprend en outre un ou plusieurs additifs choisis parmi :
- les charges, avantageusement la charge est choisie parmi les maltodextrines ; les cyclodextrines ; le lactose ; le talc ; la silice ; les silicates ; les phosphates, la poudre de cellulose ; la cellulose microcristalline ; le mica ; les carbonates
- les liants, avantageusement le liant est choisi parmi les polymères d'alcool polyvinylique, la polyvinylpyrrolidone, les copolymères de vinylpyrrolidone et d'acétate de vinyle, la carboxymethylcellulose, ses sels et ses dérivés, l'acide alginique et ses sels, la zéine, les pectines, la gomme arabique, la gomme d'acacia, la gomme adragante, la gomme karaya, la gomme xanthane, les carraghénanes, la gélatine, les polymères pullulan, les polymères agar, les amidons et leurs dérivés, les carbomères, l'acide acrylique réticulé avec des polyalkényle éthers, les polycarbophiles, et leurs mélanges
- les solvants, avantageusement le solvant est choisi parmi l'éthanol, le propylène glycol, la glycérine, l'alcool cétylique, les polyéthylène glycols et leurs dérivés, et leurs mélanges
- les arômes, avantageusement l'arôme est choisi parmi les huiles essentielles, les dérivés terpéniques tels que le menthol, et leurs mélanges
- les tensioactifs, avantageusement le tensioactif est choisi parmi les esters de glycol comme le monostéarate de glycérol, les esters d'acides gras et de sorbitan, les esters d'acides gras polyoxyéthylénés et de sorbitan ; les huiles végétales polyoxyéthylénées comme les huiles de ricin polyoxyéthylénées, les huiles végétales hydrogénées polyoxyéthylénées comme les huiles de ricin hydrogénées polyoxyéthylénées ; la lécithine et ses dérivés de soja ou d'oeuf comme la phosphatidylcholine, la phosphatidylcholine hydrogénée, la lysophosphatidylcholine, la lysophosphatidylcholine hydrogénée, ainsi que leurs mélanges
- les exhausteurs de goût, avantageusement l'exhausteur de goût est le glutamate de sodium
- les édulcorants, avantageusement l'édulcorant est choisi parmi l'aspartame ; le saccharinate de sodium ; la thaumatine ; les polyols tels que le sorbitol, le xylitol, l'isomalt, le maltitol, le mannitol, et le lactitol ; et leurs mélanges
- les anti-oxydants, avantageusement l'antioxydant est choisi parmi l'acide ascorbique, ses sels et ses dérivés, le métabisulfite de sodium ou potassium, le bisulfite de sodium, le butylhydroxyanisol, le butylhydroxytoluène, l'acide gallique et ses dérivés tel que le gallate de propyle, et leurs mélanges
- les agents de chélation, avantageusement l'agent de chélation est choisi parmi l'EDTA et ses sels, l'acide tartrique et ses sels, et leurs mélanges
- les agents conservateurs, avantageusement l'agent conservateur est choisi parmi les parabens, l'acide benzoïque, le benzoate de sodium, l'acide sorbique, le sorbate de potassium, et leurs mélanges
- les colorants, avantageusement le colorant est choisi parmi les oxydes de fer, l'oxyde de titane, le curcumin, le caramel, les carotènes, et leurs mélanges
- et les régulateurs de pH, avantageusement le régulateur de pH est choisi parmi l'acide citrique, ses sels et ses dérivés, les carbonates sodiques, la delta glucono lactone, et leurs mélanges.

3. Composition selon la revendication 2, **caractérisée en ce que** le ou les additifs représente(nt) 0,01 à 75%, de préférence 1 à 50% en poids de la composition par rapport au poids total de la composition.

4. Composition selon l'une des revendications 1 à 3, **caractérisée en ce que** la substance active est choisie parmi les anti-infectieux tels que les antibiotiques et les sulfamides, les cardiotoniques, les antiparasitaires internes et externes, les insecticides, les inhibiteurs de croissance des insectes, les anti-arthrosiques, les anti-inflammatoires stéroïdiens ou non, les antihistaminiques, les hormones telles que les prostaglandines, les substances de thérapie digestive tels que les pansements et sédatifs gastro-intestinaux, les antiulcéreux et les flores de substitution, les anti-diarrhéiques, les hépato-protecteurs, les antispasmodiques, les laxatifs, les antiseptiques intestinaux, les substances de thérapie respiratoire tels que les analeptiques respiratoires, les antitussifs, les broncho-dilatateurs, les fluidifiants bronchiques et mucolytiques, et les antiseptiques respiratoires, les substances agissant sur le système nerveux tels que les analgésiques, les sédatifs et les tranquillisants, les antiépileptiques, les anesthésiques, les orexigènes, les anorexigènes, les substances de thérapie immunitaires telles que les interleukines et l'interféron, les substances de thérapie anticancéreuses tels que les antimitotiques et les cytostatiques, les macro-, micro- et oligo-éléments, les vitamines, les extraits de plantes, les extraits d'organes d'animaux, et leurs mélanges.

5. Composition selon l'une quelconque des revendications 1 à 4, **caractérisée en ce qu'**elle comprend une substance active médicamenteuse choisie parmi les antibiotiques tels que l'amoxicilline, l'acide clavulanique, la céphalexine, la rifaximine, les antiparasitaires tels que l'ivermectine, la moxidectine, la milbémycine, le pyrantel, et ses dérivés tel que le pamoate, le praziquantel, les benzimidazoles , leurs sels ou leurs dérivés, les insecticides tel que le fampronil, les cardiotoniques tel que le levosimendan, et les anti-arthrosiques telle que la diaceréine.

6. Composition selon l'une des revendications 1 à 5, **caractérisée en ce qu'**elle comprend une substance active nutraceutique ou complément alimentaire choisie parmi les extraits de plantes, les extraits d'animaux, les flores de substitution, les macro-, micro- et oligo-éléments, les vitamines, et leurs mélanges.

7. Composition selon l'une quelconque des revendications 1 à 3 et 6, **caractérisée en ce que** la substance active nutraceutique ou complément alimentaire est choisie parmi les extraits d'animaux pour leur action anti-arthrosique tels que la chondroïtine sulfate, le chitosan et ses dérivés; pour leur action anti-ulcère et/ou anti-stress tel que l'extrait de soja fermenté ; pour leur action insecticide ou insectifuge tels que les pyrèthres, les vitamines telles que la vitamine C, la vitamine D3, les flores de substitution tel que *Enterococcus faecium,* les micro-éléments tel que le sélénium apporté par une souche de *Saccharomyces cerevisiae.*

8. Procédé de préparation d'une composition appétissante solide selon l'une quelconque des revendications 1 à 7, **caractérisé en ce qu'**il comprend :
i) un mélange des composants pulvérulents sans apport de chaleur ou d'eau,
ii) l'introduction par vaporisation des composants liquides ou préalablement liquéfiés ou préalablement dissous sous agitation jusqu'à l'obtention d'un granulat homogène, sec, fluide, ne présentant pas d'agglomérats, de prise en masse,
iii) un calibrage du granulat pour obtenir une granulation comprise entre 50 et 1000 µm, de préférence entre 200 et 600 µm, et
iv) une compression du granulat sec, fluide et calibré, avec une presse à bouillon cubes, donnant des masses compactes,
v) une mise en forme de masses compactes avec une grande homogénéité de poids.

9. Procédé de préparation d'une composition appétissante solide selon la revendication 8, **caractérisé en ce que** lesdits composants pulvérulents présentent une granulométrie inférieure à 200 µm et préférentiellement inférieure à 100 µm.

10. Procédé de préparation d'une composition appétissante solide selon l'une quelconque des revendications 8 et 9, **caractérisé en ce que** le mélange se présente sous forme d'un granulat homogène, sec, fluide de granulométrie inférieure à 600 µm.

11. Procédé de préparation d'une composition appétissante solide selon l'une quelconque des revendications 8 à 10, **caractérisée en ce que** la composition appétissante solide se présente sous forme d'une masse compacte avec au moins les surfaces supérieure et inférieure planes.

12. Procédé de préparation d'une composition appétissante solide selon l'une quelconque des revendications 8 à 11, caractérisé en ce la masse compacte présente un poids unitaire de plus ou moins de 3% de la valeur théorique du poids requis, et préférentiellement de plus ou moins de 2% de la valeur théorique du poids requis.

13. Procédé de préparation d'une composition appétissante solide selon l'une quelconque des revendications 8 à 12, **caractérisé en ce que** la presse à bouillon cubes est équipée d'un démotteur-calibreur.

14. Procédé de préparation d'une composition appétissante solide selon l'une quelconque des revendications 8 à 13, **caractérisé en ce que** la presse à bouillon cubes est équipée d'un étage de compression réfrigéré.

## Patentansprüche

1. Feste appetitanregende Zusammensetzung vom Typ Arzneimittel, Nutrazeutikum oder Nahrungsergänzung zur oralen Verabreichung an Säugetiere, Menschen ausgenommen, insbesondere für das Haustier wie den Hund, die Katze oder das Pferd, umfassend in Bezug zum Gesamtgewicht der Zusammensetzung:
- 5 bis 30 Gew.-%, vorzugsweise 8 bis 20 Gew.-%, mindestens eines Fetts, ausgewählt aus einem flüssigen Öl, einem Fettkörper, einem Wachs oder einem Gemisch derselben, wobei das flüssige Öl nicht mehr als 8 Gew.-% der Zusammensetzung darstellen darf,
- 0,001 bis 55 Gew.-% mindestens einer aktiven Substanz, und
- 20 bis 95 Gew.-%, vorzugsweise 40 bis 70 Gew.-%, mindestens eines appetitanregenden Stoffs,
**dadurch gekennzeichnet, dass**:
a) das flüssige Öl aus dem Olivenöl, dem Erdnussöl, dem Rapsöl, dem Sonnenblumenöl und ihren Gemischen ausgewählt ist;
b) der Fettkörper aus dem Hühnerfett, dem Entenfett, dem Schweineschmalz, dem Talg, der Butter, dem Palmfett, dem Palmitinstearin, der Margarine, dem eventuell gehärteten Palmöl, dem Cetylpalmitat, dem gehärteten Kokosnussöl und ihren Gemischen ausgewählt ist;
c) das Wachs aus dem Bienenwachs, dem Carnaubawachs, dem Candelillawachs und ihren Gemischen ausgewählt ist;
d) der appetitanregende Stoff aus dem Fleisch, den Fleischmehlen, den Fischmehlen, den Käsepulvern, den Milchderivaten, dem Leberpulver, der Gelatine, den Extrakten dieser tierischen Substanzen oder ihren Derivaten; der Bierhefe; den Pflanzenfasern; den pflanzlichen Produkten oder Nebenprodukten wie dem Bockshornklee, dem Apfel, der Karotte, der Futterrübe, der Zuckerrübe, dem Thymian, der Luzerne, dem Zuckerrohr, den Getreiden wie dem Hafer, dem Weizen, dem Reis und dem Mais, der Soja, ihren Derivaten wie den Mehlen und ihren Gemischen; der kristallisierten Saccharose in Pulverform, der Glucose, dem Invertzucker, der Melasse, dem Honig und seinen Derivaten und ihren Gemischen, dem Natriumchlorid ausgewählt ist, und
e) die feste Zusammensetzung durch Mischen der Bestandteile, Sprühen der Fette, Kalibrieren des trockenen und fluiden Granulats und Kompression des Granulats mit einer Bouillonwürfelpresse erhalten wird, wobei
- das Gemisch in Form eines homogenen, trockenen, fluiden Granulats vorliegt,
- das trockene und fluide Granulat eine Korngröße zwischen 50 und 1000 µm, vorzugsweise zwischen 200 und 600 µm, inklusive aufweist,
- die feste appetitanregende Zusammensetzung ein Stückgewicht von über oder unter 3 % des theoretischen Wertes des verlangten Gewichts und vorzugsweise von über oder unter 2 % des theoretischen Wertes des verlangten Gewichts aufweist.

2. Zusammensetzung nach Anspruch 1, **dadurch gekennzeichnet, dass** die Zusammensetzung ferner einen oder mehrere Zusatzstoffe umfasst, die ausgewählt sind aus:
- den Chargen, wobei die Charge aus den Maltodextrinen; den Zyklodextrinen; der Laktose; dem Talk; dem Siliziumdioxid; den Silikaten; den Phosphaten, dem Zellulosepulver; der mikrokristallinen Zellulose; dem Glimmer; den Carbonaten ausgewählt ist,
- den Bindemitteln, wobei das Bindemittel aus den Polymeren des Polyvinylalkohols, dem Polyvinylpyrrolidon, den Copolymeren von Vinylpyrrolidon und von Vinylacetat, der Carboxymethylcellulose, ihren Salzen und ihren Derivaten, der Alginsäure und ihren Salzen, dem Zein, den Pektinen, dem Gummiarabicum, dem Akaziengummi, dem Tragantgummi, dem Karayagummi, dem Xanthan, den Carrageenen, der Gelatine, den Pullulanpolymeren, den Agarpolymeren, den Stärken und ihren Derivaten, den Carbomeren, der mit Polyalkenylethern vernetzten Acrylsäure, den Polycarbophilen und ihren Gemischen ausgewählt ist,
- den Lösungsmitteln, wobei das Lösungsmittel aus dem Ethanol, dem Propylenglycol, dem Glycerin, dem Ketylalkohol, den Polyethylenglycolen und ihren Derivaten und ihren Gemischen ausgewählt ist,
- den Aromen, wobei das Aroma aus den essentiellen Ölen, den Terpenderivaten wie dem Menthol und ihren Gemischen ausgewählt ist,
- den Tensiden, wobei das Tensid in vorteilhafter Weise aus den Glycolestern wie dem Glycerolmonostearat, den Fettsäureestern und von Sorbitan, den Polyoxyethylen-Fettsäureestern und von Sorbitan; den Polyoxyethylen-Pflanzenölen wie den Polyoxyethylen-Ricinölen, den gehärteten Polyoxyethylen-Pflanzenölen wie den gehärteten Polyoxyethylen-Ricinölen; dem Lecithin und seinen Soja- oder Eiderivaten wie dem Phosphatidylcholin, dem gehärteten Phosphatidylcholin, dem Lysophosphatidylcholin, dem gehärteten Lysophosphatidylcholin sowie ihren Gemischen ausgewählt ist,
- den Geschmacksverstärkern, wobei der Geschmacksverstärker in vorteilhafter Weise Natriumglutamat ist,
- den Süßstoffen, wobei der Süßstoff in vorteilhafter Weise aus dem Aspartam; dem Natriumsaccharinat; dem Taumatin; den Polyolen wie dem Sorbitol, dem Xylitol, dem Isomalt, dem Maltitol, dem Mannitol und dem Lactitol; und ihren Gemischen ausgewählt ist,
- den Antioxidantien, wobei das Antioxidans in vorteilhafter Weise aus der Ascorbinsäure, ihren Salzen und ihren Derivaten, dem Natrium- oder Kaliummetabisulfit, dem Natriumbisulfit, dem Butylhydroxyanisol, dem Butylhydroxytoluen, der Gallussäure und ihren Derivaten wie dem Propylgallat und ihren Gemischen ausgewählt ist,
- den Chelationsmitteln, wobei das Chelationsmittel in vorteilhafter Weise aus der EDTA und ihren Salzen, der Weinsteinsäure und ihren Salzen und ihren Gemischen ausgewählt ist,
- den Konservierungsmitteln, wobei das Konservierungsmittel in vorteilhafter Weise aus den Parabenen, der Benzoesäure, dem Natriumbenzoat, der Sorbinsäure, dem Kaliumsorbat und ihren Gemischen ausgewählt ist,
- den Farbstoffen, wobei der Farbstoff in vorteilhafter Weise aus den Eisenoxiden, den Titanoxid, dem Curcumin, dem Karamell, den Carotinoiden und ihren Gemischen ausgewählt ist,
- und den pH-Regulatoren, wobei der pH-Regulator in vorteilhafter Weise aus der Citronensäure, ihren Salzen und ihren Derivaten, den Sodacarbonaten, dem Deltagluconlacton und ihren Gemischen ausgewählt ist.

3. Zusammensetzung nach Anspruch 2, **dadurch gekennzeichnet, dass** der oder die Zusatzstoffe 0,01 bis 75 Gew.-%, vorzugsweise 1 bis 50 Gew.-%, der Zusammensetzung in Bezug zum Gesamtgewicht der Zusammensetzung darstellt/darstellen.

4. Zusammensetzung nach einem der Ansprüche 1 bis 3, **dadurch gekennzeichnet, dass** die aktive Substanz aus den Antiinfektionsmitteln wie den Antibiotika und den Sulfamiden, den Kardiotonika, den Mitteln gegen innere und äußere Parasiten, den Insektiziden, den Insektenwachstumshemmern, den Antiarthrosemitteln, den steroiden oder nicht steroiden Entzündungshemmern, den Antihistaminika, den Hormonen wie den Prostaglandinen, den verdauungstherapeutischen Substanzen wie den Pflastern und den Magen-Darm-Sedativa, den Anti-Ulcus-Mitteln und den Substitutionsfloren, den Anti-Durchfall-Mitteln, den Leberschützern, den antispasmischen Mitteln, den Laxativa, den Darmantiseptika, den Atmungstherapiesubstanzen wie den Atmungsanaleptika, den Antihustenmitteln, den Bronchenerweiterern, den Bronchial-Fließmitteln und -Schleimlösern und den Atemwegs-Antiseptika, den Substanzen, die auf das Nervensystem wirken, wie die Analgetika, die Sedativa und die Beruhigungsmittel, die Antiepileptika, die Anästhetika, die Appetitanreger, die Appetitzügler, den Substanzen zur Immuntherapie wie die Interleukine und das Interferon, den Substanzen zur Antikrebstherapie wie die antimitotischen Mittel und die Zytotstatika, den Makro-, Mikro- und Spurenelementen, den Vitaminen, den Pflanzenextrakten, den Extrakten von Tierorganen und ihren Gemischen ausgewählt ist.

5. Zusammensetzung nach einem der Ansprüche 1 bis 4, **dadurch gekennzeichnet, dass** sie eine medikamentöse aktive Substanz umfasst, die aus den Antibiotika wie dem Amoxicillin, der Clavulansäure, dem Cephalexin, dem Rifaximin, den Anti-Parasiten-Mitteln wie dem Ivermectin, dem Moxidectin, dem Milbemycin, dem Pyrantel und seinen Derivaten wie dem Pamoat, dem Praziquantel, den Benzimidazolen, ihren Salzen oder ihren Derivaten, den Insektiziden wie dem Fampronil, den Kardiotonika wie dem Levosimendan und den Anti-Arthrose-Mitteln wie dem Diacerein ausgewählt ist.

6. Zusammensetzung nach einem der Ansprüche 1 bis 5, **dadurch gekennzeichnet, dass** sie eine aktive nutrazeutische Substanz oder Nahrungsergänzung umfasst, die aus den Pflanzenextrakten, den Extrakten von Tieren, den Substitutionsfloren, den Makro-, Mikro- und Spurenelementen, den Vitaminen und ihren Gemischen ausgewählt ist.

7. Zusammensetzung nach einem der Ansprüche 1 bis 3 und 6, **dadurch gekennzeichnet, dass** die aktive nutrazeutische Substanz oder Nahrungsergänzung aus den Extrakten von Tieren wegen ihrer Anti-Arthrose-Wirkung wie das Chondroitinsulfat, das Chitosan und seine Derivate; wegen ihrer Anti-Ulcus- und/oder Anti-Stress-Wirkung wie der fermentierte Sojaextrakt; wegen ihrer insektiziden oder insektifugen Wirkung wie das Pyrethrum, den Vitaminen wie das Vitamin C, das Vitamin D3, den Substitutionsfluren wie *Enterococcus faecium,* den Mikroelementen wie das Selen, bereitgestellt von einem Stamm von *Saccharomyces cerevisiae,* ausgewählt ist.

8. Verfahren zur Herstellung einer festen appetitanregenden Zusammensetzung nach einem der Ansprüche 1 bis 7, **dadurch gekennzeichnet, dass** es umfasst:
i) ein Mischen von pulverförmigen Bestandteilen ohne Zufuhr von Wärme oder Wasser,
ii) das Einleiten durch Zerstäuben der flüssigen oder zuvor verflüssigten oder zuvor durch Rühren gelösten Bestandteile bis zum Erhalt eines homogenen, trockenen, fluiden Granulats, das keine Agglomerate, Klumpen aufweist,
iii) ein Kalibrieren des Granulats, um eine Körnung zwischen 50 und 1000 µm, vorzugsweise zwischen 200 und 600 µm, inklusive zu erhalten, und
iv) ein Komprimieren des trockenen, fluiden und kalibrierten Granulats mit einer Bouillonwürfelpresse, was kompakte Massen ergibt,
v) ein Formen kompakter Massen mit einer hohen Gewichtshomogenität.

9. Verfahren zur Herstellung einer festen appetitanregenden Zusammensetzung nach Anspruch 8, **dadurch gekennzeichnet, dass** die pulverförmigen Bestandteile eine Korngröße unter 200 µm und vorzugsweise unter 100 µm aufweisen.

10. Verfahren zur Herstellung einer festen appetitanregenden Zusammensetzung nach einem der Ansprüche 8 und 9, **dadurch gekennzeichnet, dass** das Gemisch die Form eines homogenen, trockenen, fluiden Granulats mit einer Korngröße unter 600 µm hat.

11. Verfahren zur Herstellung einer festen appetitanregenden Zusammensetzung nach einem der Ansprüche 8 bis 10, **dadurch gekennzeichnet, dass** die feste appetitanregende Zusammensetzung die Form einer kompakten Masse mit mindestens einer ebenen oberen und unteren Fläche hat.

12. Verfahren zur Herstellung einer festen appetitanregenden Zusammensetzung nach einem der Ansprüche 8 bis 11, **dadurch gekennzeichnet, dass** die kompakte Masse ein Stückgewicht von über oder unter 3 % des theoretischen Wertes des verlangten Gewichts und vorzugsweise von über oder unter 2 % des theoretischen Wertes des verlangten Gewichts hat.

13. Verfahren zur Herstellung einer festen appetitanregenden Zusammensetzung nach einem der Ansprüche 8 bis 12, **dadurch gekennzeichnet, dass** die Bouillonwürfelpresse mit einer Hebe-Kalibrier-Vorrichtung ausgestattet ist.

14. Verfahren zur Herstellung einer festen appetitanregenden Zusammensetzung nach einem der Ansprüche 8 bis 13, **dadurch gekennzeichnet, dass** Bouillonwürfelpresse mit einer gekühlten Kompressionsstufe ausgestattet ist.

## Claims

1. A solid, palatable composition as medicinal product, nutraceutical or food supplement, for oral administration to mammals, except human, in particular to domestic animals such as dogs, cats or horses comprising relative to the total weight of the composition:
- 5 to 30 %, preferably 8 to 20 % by weight of at least one fat chosen from among a liquid oil, a fat, a wax or mixture thereof, the liquid oil not to represent more than 8 % by weight of the composition,
- 0.001 to 55 % by weight of at least one active substance, and
- 20 to 95 %, preferably 40 to 70 % by weight of at least one palatable material,
**characterized in that**:
a) the liquid oil is chosen from among olive oil, groundnut oil, rapeseed oil, sunflower oil, and the mixtures thereof;
b) the fat is chosen from among chicken fat, duck fat, lard, tallow, butter, palm fat, palm stearin, margarine, palm oil optionally hydrogenated, cetyl palmitate, hydrogenated coconut oil, and the mixtures thereof;
c) the wax is chosen from among beeswax, carnauba wax, candelilla wax, and the mixtures thereof;
d) the palatable material is chosen from among meat, meat powders, fish powders, cheese powders, milk derivatives, liver powder, gelatine, the extracts of these animal substances or their derivatives; beer yeast; vegetable fibres; vegetable products and by-products such as fenugreek, apple, carrot, fodder beet, sugar beet, thyme, alfalfa, sugar cane, cereals such as oats, wheat, rice, corn, soy, their derivatives such as flours and the mixtures thereof; crystallised sucrose, powdered, glucose, invert sugar, molasses, honey and its derivatives, and the mixtures thereof, sodium chloride, and
e) said solid composition is obtained by mixing the components, vaporising the fats, calibrating the dry, fluid granular material and compressing the granular material in a stock cube press, wherein
- the mixture is in the form of a dry, fluid, homogeneous granular material,
- the dry, fluid granular material has a particle size of between 50 and 1000 µm, preferably between 200 and 600 µm,
- the solid, palatable composition has a unit weight to within about 3% of the theoretical value of the required weight, and preferably to within about 2% of the theoretical value of the required weight.

2. The composition according to claim 1, **characterized in that** the composition further comprises one or more additives chosen from among:
- fillers, advantageously the filler is chosen from among maltodextrins; cyclodextrins; lactose; talc; silica; silicates; phosphates, cellulose powder; microcrystalline cellulose; mica; carbonates,
- binders, advantageously the binder is chosen from among polyvinyl alcohol polymers, polyvinylpyrrolidone, the copolymers of vinylpyrrolidone and vinyl acetate, carboxymethylcellulose, its salts and derivatives, alginic acid and its salts, zein, pectins, gum arabic, acacia gum, gum tragacanth, karaya gum, xanthan gum, carrageenans, gelatine, pullulan polymers, agar polymers, starches and their derivatives, carbomers, acrylic acid cross-linked with polyalkenyl ethers, polycarbophils, and the mixtures thereof,
- solvents, advantageously the solvent is chosen from among ethanol, glycol propylene, glycerine, cetyl alcohol, polyethylene glycols and their derivatives, and the mixtures thereof,
- flavourings, advantageously the flavouring is chosen from among essential oils, terpene derivatives such as menthol, and the mixtures thereof,
- surfactants, advantageously the surfactant is chosen from among glycol esters such as glycerol monostearate, the esters of fatty acids and sorbitan, the esters of polyoxyethylenated fatty acids and sorbitan; polyoxyethylenated vegetable oils such as polyoxyethylenated castor oils, polyoxyethylenated hydrogenated vegetable oils such as polyoxyethylenated hydrogenated castor oils; lecithin and its soy or egg derivatives such as phosphatidylcholine, hydrogenated phosphatidylcholine, lysophosphatidylcholine, hydrogenated lysophosphatidylcholine, and the mixtures thereof,
- taste enhancers, advantageously the taste enhancer is sodium glutamate,
- sweeteners, advantageously the sweetener is chosen from among aspartame; sodium saccharin; thaumatin; polyols such as sorbitol, xylitol, isomalt, maltitol, mannitol and lactitol; and the mixtures thereof,
- antioxidants, advantageously the antioxidant is chosen from among ascorbic acid, its salts and derivatives, sodium or potassium metabisulphite, sodium bisulphite, butylhydroxyanisol, butylhydroxytoluene, gallic acid and its derivatives such as propyl gallate, and the mixtures thereof,
- chelating agents, advantageously the chelating agent is chosen from among EDTA and its salts, tartaric acid and its salts, and the mixtures thereof.
- preserving agents, advantageously the preserving agent is chosen from among parabens, benzoic acid, sodium benzoate, sorbic acid, potassium sorbate, and the mixtures thereof,
- colouring agents, advantageously the colouring agent is chosen from among iron oxides, titanium oxide, curcumin, caramel, carotenes, and the mixtures thereof, and
- pH regulators, advantageously the pH regulator is chosen from among citric acid, its salts and derivatives, sodium carbonates, delta glucono lactone, and the mixtures thereof.

3. The composition according to claim 2, **characterized in that** the additive(s) represent 0.01 to 75 %, de preferably 1 to 50 % by weight of the composition relative to the total weight of the composition.

4. The composition according to one of claims 1 to 3, **characterized in that** the active substance is chosen from among anti-infectives such as antibiotics and sulfonamides, cardiotonics, internal and external anti-parasitics, insecticides, insect growth inhibitors, anti-arthritics, anti-inflammatories whether or not steroidal, anti-histaminics, hormones such as prostaglandins, substances for digestive therapy such as gastro-intestinal dressings and sedatives, anti-ulcer agents and substitution flora, anti-diarrhoeals, hepato protectors, antispasmodics, laxatives, intestinal antiseptics, substances for respiratory therapy such as respiratory analeptics, antitussives, bronchodilators, bronchial and mucolytic fluidifiers and respiratory antiseptics, substances acting on the nervous system such as analgesics, sedatives and tranquillisers, anti-epileptics, anaesthetics, orexigenics, anorexigenics, substances for immunity therapy such as interleukins and interferon, substances for anticancer therapy such as antimitotics and cytostatics, macro-, micro-nutrients and trace elements; vitamins, plant extracts, extracts from animal organs, and the mixtures thereof.

5. The composition according to any one of claims 1 to 4, **characterized in that** it comprises an active medicinal substance chosen from among antibiotics such as amoxicillin, clavulanic acid, cephalexin, rifaximin, anti parasitics such as ivermectin, moxidectin, milbemycin, pyrantel and its derivatives such as the pamoate, praziquantel, benzimidazoles, their salts or derivatives, insecticides such as fampronil, cardiotonics such as levosimendan, and anti-arthritics such as diacerein.

6. The composition according to one of claims 1 to 5, **characterized in that** it comprises an active nutraceutical or food supplement substance chosen from among plant extracts, animal extracts, substitution flora, macro-, micro-nutrients and trace elements, and the mixtures thereof.

7. The composition according to any one of claims 1 to 3 and 6, **characterized in that** the active nutraceutical or food supplement substance is chosen from among animal extracts for their anti-arthritic action such as chondroitin sulfate, chitosan and its derivatives; for their anti-ulcer and/or anti stress action such as fermented soy extract; for their insecticidal or insect repellent action such as pyrethrums, vitamins such a vitamin C, vitamin D3, substitution flora such as *Enterococcus faecium,* micro-nutrients such as selenium provided by a strain of *Saccharomyces cerevisiae.*

8. A process for preparing a solid, palatable composition according to any one of claims 1 to 7, **characterized in that** it comprises:
i) mixing the powder components without the addition of heat or water,
ii) by vaporisation, adding the liquid or previously liquefied or previously dissolved components under stirring until a dry, fluid homogeneous granular material is obtained having no agglomerates and no clumping,
iii) calibrating the granular material to obtain a particle size of between 50 and 1000 µm, preferably between 200 and 600 µm, and
iv) compressing the calibrated, dry, fluid granular material in a stock cube press to obtain compact masses,
v) shaping the compact masses with high weight homogeneity.

9. The process for preparing a solid, palatable composition according to claim 8, **characterized in that** said powder components have a particle size of less than 200 µm and preferably less than 100 µm.

10. The process for preparing a solid, palatable composition according to any one of claims 8 and 9, **characterized in that** the mixture is in the form of dry, fluid homogeneous granular material having a particle size of less than 600 µm.

11. The process for preparing a solid, palatable composition according to any one of claims 8 to 10, **characterized in that** the solid, palatable composition is in the form of a compact mass of which at least the top and bottom surfaces are planar.

12. The process for preparing a solid, palatable composition according to any one of claims 8 to 11, **characterized in that** the compact mass has a unit weight to within about 3% of the theoretical value of the required weight, and preferably to within about 2% of the theoretical value of the required weight.

13. The process for preparing a solid, palatable composition according to any one of claims 8 to 12, **characterized in that** the stock cube press is equipped with a cluster-breaker/size grader.

14. The process for preparing a solid, palatable composition according to any one of claims 8 to 13, **characterized in that** the stock cube press is equipped with a chilled compression stage.
